Europäisches Patentamt

European Patent Office

Office européen des brevets

(11) Publication number: **0 126 805**
**B1**

## EUROPEAN PATENT SPECIFICATION

(45) Date of publication of patent specification: **17.08.88**

(21) Application number: **83112335.1**

(22) Date of filing: **26.06.80**

(60) Publication number of the earlier application in accordance with Art. 76 EPC: **0 042 889**

(51) Int. Cl.⁴: $A\ 61\ N\ 1/42$, $A\ 61\ B\ 17/56$, $H\ 01\ F\ 5/00$

(54) Electromagnetic body-treatment device.

(43) Date of publication of application:
**05.12.84 Bulletin 84/49**

(45) Publication of the grant of the patent:
**17.08.88 Bulletin 88/33**

(84) Designated Contracting States:
**BE CH DE FR GB IT LI LU NL SE**

(56) References cited:
**US-A-3 915 151**
**US-A-4 066 065**
**US-A-4 105 017**
**US-A-4 186 729**

(73) Proprietor: **ELECTRO-BIOLOGY, INC**
**300 Fairfield Road**
**Fairfield New Jersey 07006 (US)**

(72) Inventor: **Ryaby, John P.**
**36 Maple Lane**
**Essex Fells New Jersey 07021 (US)**
Inventor: **Pilla, Arthur A.**
**371 Martom Road**
**Wyckoff New Jersey 07481 (US)**

(74) Representative: **Wright, Peter David John et al**
**R.G.C. Jenkins & Co. 26 Caxton Street**
**London SW1H 0RJ (GB)**

Courier Press, Leamington Spa, England.

## Description

The invention concerns an electromagnetic body treatment device.

U.S. Patent No. 4,l05,017 describes a method and apparatus for the treatment of living tissues and/or cells by altering their interaction with the charged species in their environment. In particular, as described in this U.S. Patent, which is incorporated herein by reference, a controlled modification of cellular and/or tissue growth, repair and maintenance behaviour may be achieved by the application of encoded electrical information. Still more particularly, the treatment involves the application by a surgically non-invasive direct inductive coupling, of one or more electrical voltage and concomitant current signals conforming to a highly specific pattern.

Several attempts have been made in the past to elicit a response of living tissue to electrical signals.

Investigations have been conducted involving the use of direct current, alternating current, and pulsed signals of single and double polarity. Invasive treatments involving the use of implanted electrodes have been followed, as well as non-invasive techniques utilizing electrostatic and electromagnetic fields. Much of the prior work that has been done is described in Volume 238 of the Annals of The New York Academy of Sciences published 11 October, 1974 and entitled "Electrically Mediated Growth Mechanisms in Living Systems" (Editors A. R. Liboff and R. A. Rinaldi). See also "Augmentation of Bone Repair by Inductively Coupled Electromagnetic Fields" by C. Andrew L. Bassett, Robert J. Pawluk and Arthur A. Pilla published in Volume 184, pages 575-577 of Science (3rd May, 1974).

The invention of U.S. Patent No. 4,l05,017 is based upon basis cellular studies and analyses which involve a detailed consideration of the interactions of charged species, such as divalent cations and hormones at a cell's interfaces and junctions.

Basically, it has been established that, by changing the electrical and/or electrochemical environment of a living cell and/or tissue, a modification, often a beneficial therapeutic effect, of the growth, repair and maintenance behaviour of said tissue and/or cells can be achieved. This modification or effect is carried out by subjecting the desired area of tissues and/or cells to a specifically encoded electrical voltage and concomitant current, whereby the interactions of charged species at the cells' surfaces are modified. Such modifications engender a change in the state or function of the cell or tissue which may result in a beneficial influence on the treated site. For example, in the specific case of bone growth and repair, it is possible with one electrical code, hereinafter referred to as Mode 1, to change the interaction of the ion such as $Ca^{2+}$ with a cell's membranes. Whereas, with another electrical code, hereinafter referred to as Mode 2, a modification in the same cell's protein-synthesis capabilities can be affected.

For example, tissue-culture experiments involving the study of embryonic chick-limb rudiments show that the use of a Mode 1 code signal elicits enchanced $Ca^{2+}$ release of up to 50% from the competent osteogenic cell. This effect is highly specific to the parameters of the electrical code of Mode I. Thus, this code influences one major step of ossification, i.e., the mineralization of a bone-growth site. Similar tissue-culture studies using Mode 2 code signals have demonstrated that this code is responsible for enhanced protein production from similar competent osteogenic cells. This latter effect is also highly specific to the parameters of the electrical code of Mode 2. In other words, this code affects certain metabolic processes for these types of cells such as those involved in calcium uptake or release from mitochrondria as well as the synthesis of collagen, a basic structural protein of bone.

These studies show that the electrical codes of Mode 1 and Mode 2 elicit individual tissue and cellular responses, indicating that each code contains a highly specific informational content therein. Based upon these and other studies, it has been possible to utilize Mode 1 or Mode 2 signals or a particular combination of Mode 1 and Mode 2 signals to achieve a specific response required to enable the functional healing of a bone disorder. These electrical modes have been applied successfully to human and animal patients for non-healing fractures such as congenital pseudarthrosis and non-unions as well as fresh fractures. Successes achieved in the congenital pseudarthrosis cases are particularly noteworthy, since normally 80% of children thus afflicted required amputation, since conventional treatments such as bone grafting and internal fixation are unsuccessful.

While there have been previously many investigations of the response of living tissue and/or cells to electrical signals, clinical results prior to the date of the above U.S. Patent using earlier techniques have not been uniformly successful or generally accepted within the appropriate professional community. Several reasons contributed to this state. First, it had not been realised that electrical signals of very specific informational content were required to achieve a specifically desired beneficial clinical effect on tissue and/or cells. Second, most of the earlier techniques utilized implanted electrodes, which by virtue of unavoidable faradaic (electrolysis) effects are often more toxic than beneficial in the treated site. Furthermore, the cells and/or tissues were subjected to a highly uncontrolled current and/or voltage distribution, thereby compromising the ability of the cells to respond, should they do so, to the applied signal. This highly uncontrolled and/or voltage distribution also applied in the case of capacitatively coupled signals.

In contrast, the surgically non-invasive direct inductive coupling of electrical informational content of specific electrical codes as described in U.S. Patent No. 4,l05,017 produces within living tissue and/or cells a controlled response.

The techniques described in that patent involve the recognition that the growth, repair and maintenance behaviour of living tissue and/or cells can be modified beneficially by the application thereto of a specific electrical information. This is achieved by applying pulse waveforms of voltage and concomitant current of specific time-frequency-amplitude relations to tissue and/or cells by a surgically non-invasive means through use of a varying electromagnetic field which is inductively coupled through direct induction into or upon the tissue and/or cells under treatment.

The information furnished to the cells and/or tissues by these signals is designed to influence the behaviour of non-excitable cells such as those involved in tissue growth, repair, and maintenance. These growth, repair and maintenance phenomena are substantially different from those involved in excitable cellular activity (e.g., nerves, muscles, etc.,), particularly with respect to the type of perturbation required. Thus, the voltages and concomitant currents impressed on the cells and/or tissues are at least three orders of magnitude lower than those required to effect cellular activities such as cardiac pacing, bladder control etc.

The invention provides a treatment device for use in the treatment described in U.S. Patent No. 4,l05,017.

According to one aspect of the invention, there is provided an electromagnetic body-treatment device for surgically non-invasive modification of the growth, repair and maintenance behaviour of living tissues and cells by a specific and selective change in electrical environment, comprising electrical coil means, a rigid tubular frame carrying said coil means for supporting said coil means in relation to an afflicted body region to be treated, said coil means having turns about a flux-development axis through a treatment zone for receiving the afflicted body region, and pulse-generator means for electrically exciting said coil means with a succession of low-voltage pulses, characterised in that said rigid tubular frame is formed with an opening in its tubular wall whereby a body member having the afflicted region may be removably entered into said frame member and pass through the frame opening to place the afflicted region in the treatment zone of said coil means.

According to another aspect of the invention, there is provided an electromagnetic body-treatment device for surgically non-invasive modifi-·cation of the growth, repair and maintenance behaviour of living tissues and cells by a specific and selective change in electrical environment, comprising electrical coil means, retaining means for supporting said coil means in relation to an afflicted body region to be treated, and pulse generator means for electrically exciting said coil means with a suooession of low-voltage pulses, characterised in that said retaining means comprises a longitudinally split tube of compliant non-magnetic material which is transiently deformable in the course of assembly to a body member to be treated and which when thus assembled is circumferentially continuous, and in that said coil means comprises two like coil-winding sub-assemblies each of which is adapted for end assembly to the respective ends of said tube, the assembled spacing of said coil-winding sub-assemblies being less than their diameter, and flexible means electrically connecting said coil-winding sub-assemblies for concurrent exciation in flux-aiding relation.

The invention together with other features thereof (as set forth with particularity in the appended claims) will be more completely understood by reference to the following detailed description in conjunction with the accompanying drawings in which:

Figs. 1 and 2 are simplified views in perspective showing exemplary body-treatment devices in accordance with the present invention intended for particular purposes;

Fig. 3 is a diagram to illuminate discussion of dual-coil placement considerations;

Figs. 4, 5 and 6 are similar pairs of views a and b, respectively schematically representing front and side elevational views for each of three different generally elliptical dual-coil configurations; and

Figs. 7 to 9 are views similar to Figs. 1 and 2 to show coil arrangements for further body treatment devices in accordance with the present invention.

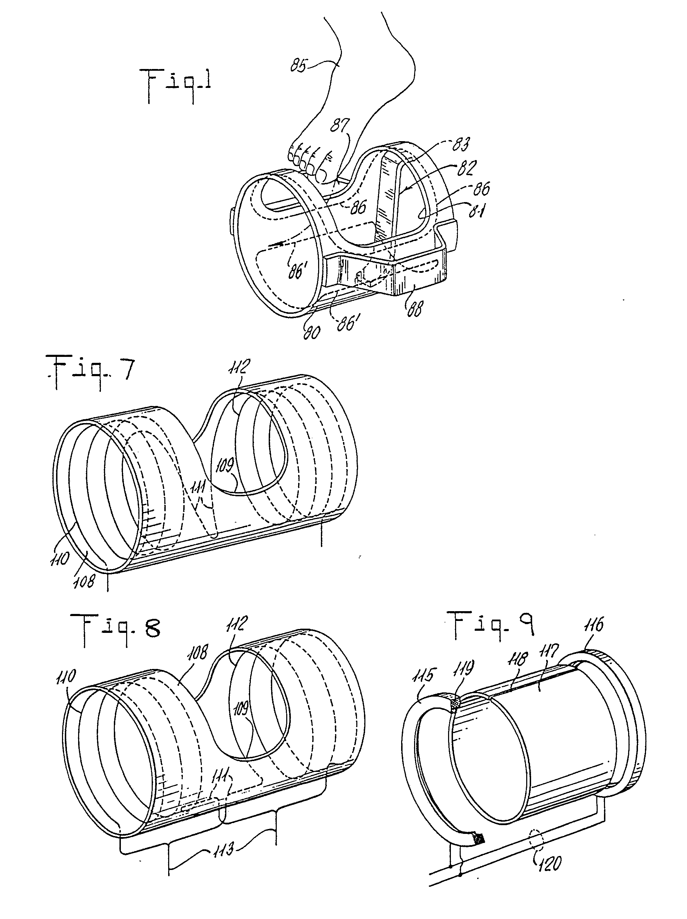

As described in US-A-4105017 aforementioned, the methods and apparatus described therein are susceptible of modification. For example, while Figs. 1 and 2 of US-A-4105017 illustrate a treatment unit which may be strapped to the leg, it is said in US-A-4105017 that treatment units incorporated in casts, e.g., may be employed. Further, treatment may be carried out by use of one or more coils of varying shapes positioned adjacent to tissue and/or cells to be treated. In fact, some treatments of humans have involved coils positioned upon opposite sides of a bone break. Coils with metal cores may also be used. In the case of treatment within the oral cavity, it is believed that double coils are advantageous, positioned, for example, on opposite, sides of a tooth socket to stimulate repair of that socket. The present invention is concerned with the provision of improved treatment units particularly though not exclusively for use in the performance of the methods disclosed in US-A-4105017.

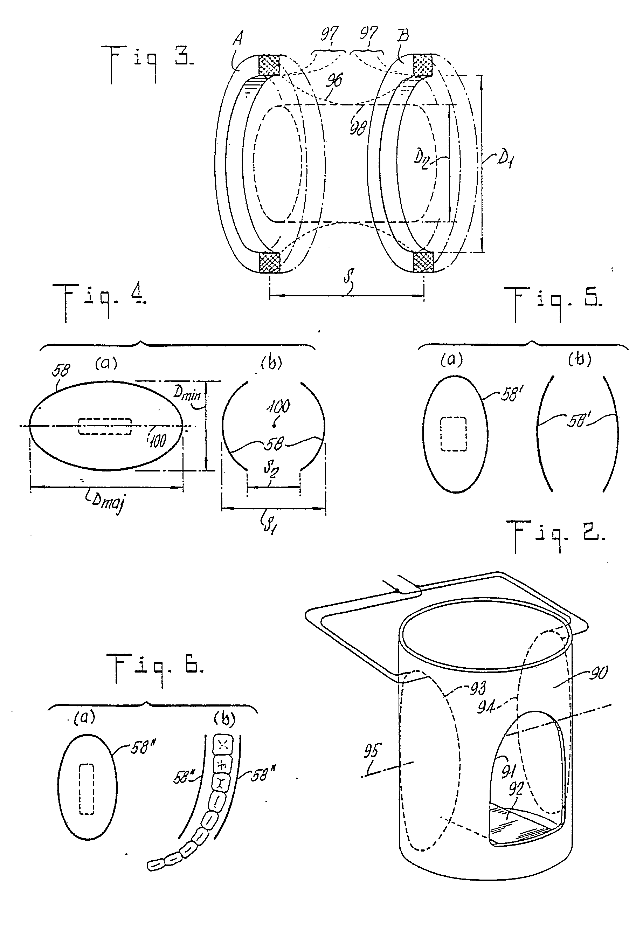

Fig. 1 depicts a body-treatment device according to the present invention which is particularly suited to the treatment of bone affliction in the region of the heel. For simplicity in Fig. 1, the showing is limited to relatively rigid structural components, and the foamed-plastic lining carried by such structure for patient comfort (i.e., to avoid chafing) has been omitted. Basically, the rigid structure of Fig. 1 comprises a tubular shell 80, as of methylmethacrilate, being open at its longitudinal ends and locally open at 81, over an angular span (about the shell axis) and intermediate the longitudinal ends of shell 80. An "S"-shaped strap 82, which may be of the same material as shell 80, has its upper end secured at 83 to the back end of shell 80, at opening 81, and its lower end 84 extends along the diametrically opposite region of the inner surface of shell 80, to define a plate for basic support of the bottom of a foot 85, to be inserted via the opening 81. The respective courses of two arcuately curved elliptical coils 86-86' are schematically indicated by heavy dashed lines. These coils will be understood to be bonded to shell 80 in vertically opposed relation, the upper coil 86 being bonded to the inner surface of shell 80, just inside the edge of opening 81, and the lower coil 86' being similarly bonded at the diametrically opposite location. Coils 86-86' thus have a permanent relation to each other and it will be understood that coils 86-86' are preferably electrically connected in parallel, in flux-aiding polarity, and adapted to be excited for example by one or the other of the energizing circuits of Figs. 4 and 5 of US-A-4105017.

In addition to the described coil-positioning and foot-supporting structure, the device of Fig. 1 includes side-bumper guards 8788 which may be bowed strips of the same plastic material as shell 80 suitably bonded at both ends to the respective longitudinal ends of shell 80. Strips 87-88 are preferably stiffly yieldable, to cushion the treated region from mechanical shock in the event of unwitting contact with furniture or other objects.

Fig. 2 is a simplified diagram similar to Fig. 1 to illustrate another body-treatment device according to the present invention, configured for application to an afflicted ankle region, or to a lower tibia/femur region. Again, the basic rigid structure is seen to comprise a tubular shell 90, as of suitable plastic. A single local side-wall opening 91 in shell 90 has a straight lower edge, contiguous to a bottom plate or rest 92 which diametrically spans the lower end of shell 90. Opposed electrical coils 93-94 are bonded to the inner surface of shell 90 at an elevation such that the alignment 95 of their centers of symmetry will geometrically intersect the center of the afflicated region, preferably as confirmed by X-ray observation on the alignment 95. The configuration of coils 93-94 may be circular or elliptical, but is preferably cylindrically arcuate, in conformance with the local shell surface to which each of them is bonded; in the event of elliptical coil configurations, the major-axis orientation is preferably vertical. Interconnection and excitation of coils 93-94 may be as described for the above-described device of Fig. l.

The described devices represent major advances in surgically non-invasive treatment of body cells, particularly as they may be involved in bone repair and healing. With respect to the body-treatment devices which have been described, we have not yet established the full range of dimensional limitations, but certain beneficial ranges can be described in general terms, particularly for the dual-coil embodiments illustratively disclosed in connection with Figs. 1 and 2.

On the elemental basis, it is convenient to consider the circular-coil situation depicted in Fig. 3, wherein like circular coils A-B of inside diameter $D_1$ are positioned on a common central axis of symmetry, at parallel planes which are spaced apart by the distance S, and wherein the coils A-B are excited in flux-aiding relation. If the spacing S is sufficiently small in relation to the diameter $D_1$, then substantially all flux lines within coils A-B will extend continuously therebetween, on a generally straight alignment which may even neck down as suggested by the profile 96. If the spacing S is greater (again in relation to the diameter $D_1$), some stray-flux lines 97 will develop, to the detriment of the development of uniform high-density flux in the central span S. Generally, in view of the necking down (96), and in view of the treatment zone being generally at the center of span S, it is convenient to consider the coils A-B as being desirably effective in producing the uniform flux distribution over an imaginary cylinder 98 of diameter $D_2$, tangent to the neck-down profile 96. From our experience to date, we can state that for body application of the character presently described, the span S should be equal to or less than the diameter $D_1$, and of course $D_2$ (the effective diameter of the zone of body treatment) will always be considerably less than $D_1$, being substantially equal to $D_1$ only when coils A-B are closely adjacent.

As a practical consideration in the application of dual coils to the body, we consider that the nominal inside diameter $D_1$ of the coils should be at least l.5 times the diameter $D_2$ of the effective body-treatment zone, and this has been found to be a reliable approach for coil spacing S substantially equal to the inside diameter $D_1$.

Having thus considered criteria factors for the simplified case of flat circular coils, it is possible to develop general criteria applicable to elliptical coils which are "wrapped" in general conformance with a cylindrical arc. Fig. 4a and 4b schematically depict a coil-58 relationship similar to that of Fig. 2, wherein the cylindrical arc of "wrapped" coil curvature is about a central axis 100, which is parallel to the major axis of the coil ellipse. And Figs. 5a and 5b schematically depict a coil-58' relationship wherein the cylindrically arcuate curvature of the coils is parallel to the minor axis of each coil. In both cases, the typical resultant treatment-zone section is suggested by dashed outline in the front view (Fig. 4a and Fig. 5a).

For purposes of deducing central magnetic-field distribution between opposed coils 58, their major-axis regions may be deemed to be at maximum separation $S_1$ and their minor-axis regions may be deemed to be at minimum separation $S_2$, as viewed in Fig. 4b. This being the case, major-axis-region contributions to the magnetic field may be deemed to apply for the span S (of Fig. 3) equal to $S_1$ (of Fig. 4b) in the context of an effective inside diameter $D_{maj.}$ which corresponds to the major axis of the ellipse; by the same token, minor-axis-region contributions to the magnetic field may be deemed to apply for a span $S_2$ (of Fig. 5b) in the context of an effective inside diameter $D_{min.}$ which corresponds to the minor axis of the ellipse. For sectional considerations at planes intermediate those of the major axis and of the minor axes, the field will follow distribution considerations intermediate those controlling distribution in planes of the major axes and of the minor distribution in planes of the major axes and of the minor axes, respectively.

Reasoning applied above as to magnetic-field distribution for the Fig. 4 configuration can also be applied to that of Fig. 5, except of course that patterns will differ by reason of the cylindrical curvature about an axis parallel to the minor elliptical axis.

The arrangement of Figs. 6 and 6b schematically depicts an embodiment of the invention which uses two generally cylindrically arcuate coils 58″ wherein the cylindrical arcs are nested in spaced relation appropriate to the desired application, electrical connection being again understood to be for flux-aiding. The coil arrangement of Figs. 6a and 6b will be understood to have application over a generally cylindrically arcuate treatment zone, as for example in the case of a jaw segment or group of teeth, the latter being suggested schematically at 101 in Fig. 6b. Depending upon the size of coils 58″, it will be understood that they may be retained in fixed spacing using a suitable bracket which bridges only teeth in the case of insertion of both coils in the mouth, and which bridges teeth as well as the adjacent cheek (via the mouth) in the case of one coil inside and the other coil outside the mouth. It will also be understood that the purposes of certain desired flux distribution within the mouth, as for dental and/or jaw osteogenesis, the inner coil 58″ may be of smaller physical size than the outer coil 58″.

It will be understood that the foregoing discussion of general principles is with a view to illustration and not limitation, and that modifications may be made without departing from the scope of the invention. For example, if for certain purposes it is not possible to construct both coils of a dual-coil embodiment so as to completely match in geometry and electrical properties, as suggested above for a dental or jaw application, there can still be a useful employment of the invention, using magnetic-flux distribution which may not be as uniform as discussed in the foregoing but which nevertheless derives benefit from the flux-aiding co-operation of two coils in opposite sides of the afflicted region under treatment, such benefit flowing of course from the excitation of such coils by the specially characterized inputs discussed in US-A-4105017.

Figs. 7 to 9 are concerned with coil configurations applicable to flux development along and therefore generally parallel to the longitudinal direction of a body member to be treated.

In Fig. 7, a single winding is shown carried by one of the cylindrical surfaces of a tubular member 108, and the latter is locally cut at an opening 109 (as in the manner described at 81 in Fig. 1) to permit insertion of a joint region such as the elbow, with the forearm projecting out one axial end of tubular member 108, and with the upper arm projecting radially outward via opening 109. The single winding is shown as a first plurality 110 of helical turns continuously connected by an axially expanded turn 111 to a second plurality 112 of similar turns, the pluralities 110-112 being positioned on opposite longitudinal sides of the opening 109 and at a spacing which is at least no greater than the effective diameter of the turns 110-112.

The arrangement of Fig. 8 is similar to that of Fig. 7 except that the respective pluralities of turns 110-112 are electrically connected in parallel, in flux-aiding relation. A central access port will be understood to be provided in tubular member 108 at a location opposite the opening 109, to permit excitation wiring connections to be provided external to all turns, i.e., no supply lines passing within any of the turns at 110-112.

In the arrangement of Fig. 9, two coil subassemblies 115-116 are constructed for assembly to the respective ends of a longitudinally split compliant-supporting member 117 of non-magnetic material. The longitudinal split at 118 permits a degree of flexibility in application to a body member, as for example during the course of its assembly past the heel region to a leg part to be treated. Each of the coil sub-assemblies is shown to be a relatively rigid annular assembly of a winding to a potting of cured hardenable material, and formed with a counterbore 119 at which the coil sub-assembly is telescopically assembled over the end of the adjacent end of tubular member 117. The inner end of each counterbore defines an inward flange to limit coil assembly, and to determine repeatably accurate spaced retention of the two coil sub-assemblies. Electrical connections to the coil sub-assemblies are shown to be parallel, and should be in flux-aiding relation, and a flexible-cable interconnection is suggested at 120.

It will be understood that various simplifying techniques have been adopted to make for more readily understood reference to the drawings. For example, in the rigid-frame coil-supporting embodiments it will be understood that in application to the body certain cushioning liner materials such as urethane foam are preferably adhered to the described structure for comfortable engagement with the body at the region of application, but to have shown such liners would only encumber the drawings.

**Claims**

1. An electromagnetic body-treatment device for surgically non-invasive modification of the growth, repair and maintenance behaviour of living tissues and cells by a specific and selective change in electrical environment, comprising electrical coil means (86,86';93,94;110,111,112), a rigid tubular frame (80;90;108) carrying said coil means for supporting said coil means in relation to an afflicted body region to be treated, said coil means having turns about a flux-development axis through a treatment zone for receiving the afflicted body region, and pulse-generator means for electrically exciting said coil means with a succession of low voltage pulses, characterised in that said rigid tubular frame is formed with an opening (81;91;l09) in its tubular wall whereby a body member having the afflicted region may be removably entered into said frame member and pass through the frame opening to place the afflicted region in the treatment zone of said coil means.

2. A treatment device according to claim 1, characterised in that said coil means comprises two coils (86, 86') mounted at diametrically opposed locations of said tubular frame, and in that said opening is located within one of said coils.

3. A treatment device according to claim 1, characterised in that said coil means comprises two coils (93,94) mounted at diametrically opposed locations of said tubular frame, and in that said opening is located intermediate said coils.

4. A treatment device according to claim 2 or claim 3, characterised in that each of said coils is of generally elliptically configuration.

5. A treatment device according to claim 1, characterised in that said coil means comprises at least one coil winding having a plurality of turns generally helically developed along the axis of said tubular frame, in that said plurality of turns are arranged as axially spaced helices (110,112) with the spacing between said helices exceeding the effective diameter of said turns, and in that said opening (109) is located between said helices.

6. An electromagnetic body-treatment device for surgically non-invasive modification of the growth, repair and maintenance behaviour of living tissues and cells by a specific and selective change in electrical environment, comprising electrical coil means (115, 116), retaining means (117) for supporting said coil means in relation to an afflicted body region to be treated, and pulse generator means for electrically exciting said coil means with a succession of low-voltage pulses, characterised in that said retaining means comprises a longitudinally split tube (117) of compliant non-magnetic material which is transiently deformable in the course of assembly to a body member to be treated and which when thus assembled is circumferentially continuous, and in that said coil means comprises two like coil-winding sub-assemblies each of which is adapted for end assembly to the respective ends of said tube, the assembled spacing of said coil-winding sub-assemblies being less than their diameter, and flexible means (120) electrically connecting said coil-winding sub-assemblies for concurrent excitation in flux-aiding relation.

7. A treatment device according to claim 6, characterised in that each of said coil-winding sub-assemblies comprises a plurality of winding turns embedded in a cured annular body of non-magnetic hardenable material having a counterbore at one end for telescoped assembly over an end of said tube, the inner end of the counterbore defining a flange to limit the telescoped assembly upon abutment with the tube end.

**Patentansprüche**

1. Elektromagnetische Körperbehandlungsvorrichtung zur operativ nicht-invasiven Modifikation des Wachstums-, Wiederherstellungs- und Erhaltungsverhaltens von lebenden Geweben und Zellen durch eine spezifische sowie selektive Änderung in einer elektrischen Umgebung mit einer elektrischen Spuleneinrichtung (86, 86'; 93, 94; 110, 111, 112), mit einem starren, rohrförmigen Stützmantel (80; 90; 108), der die Spuleneinrichtungen zur Abstützung der Spuleneinrichtungen in bezug auf eine erkrankte, zu behandelnde Körpergegend trägt, wobei die Spuleneinrichtungen Windungen um eine Kraftflußentwicklungsachse durch eine die erkrankte Körpergegend aufnehmende Behandlungszone hindurch aufweisen, und mit einer Impulserzeugereinrichtung, die die Spuleneinrich tungen elektrisch mit einer Folge von Niederspannungsimpulsen erregt, dadurch gekennzeichnet, daß der starre, rohrförmige Stützmantel mit einer Öffnung (81; 91; 109) in seiner Rohrwand versehen ist, so daß ein die erkrankte Gegend enthaltender Körperteil herausnehmbar in den Stützmantel eingeführt und durch die Mantelöffnung treten kann, um die erkrankte Gegendin der Behandlungszone der Spuleneinrichtungen zu plazieren.

2. Behandlungsvorrichtung nach Anspruch 1, dadurch gekennzeichnet, daß die Spuleneinrichtungen zwei an diametral entgegengesetzten Stellen des rohrförmigen Stützrahmens angebrachte Spulen (86, 86') umfassen und daß die Öffnung innerhalb einer der Spulen angeordnet ist.

3. Behandlungsvorrichtung nach Anspruch 1, dadurch gekennzeichnet, daß die Spuleneinrichtungen zwei an diametral entgegengesetzten Stellen des rohrförmigen Stützrahmens angebrachte Spulen (93, 94) umfassen und daß die Öffnung zwischen diesen Spulen liegend angeordnet ist.

4. Behandlungsvorrichtung nach Anspruch 2 oder Anspruch 3, dadurch gekennzeichnet, daß jede der Spulen von allgemein elliptischer Gestalt ist.

5. Behandlungsvorrichtung nach Anspruch 1, dadurch gekennzeichnet, daß die Spuleneinrichtungen wenigstens eine Spulenwicklung mit einer Mehrzahl von allgemein schraubenförmig längs der Achse des rohrförmigen Stützmantels gewikkelten Windungen aufweisen, daß die in Mehrzahl vorhandenen Windungen als axial beabstandete Wendel (110, 112) angeordnet sind, wobei der Abstand zwischen den Wendeln über den effektiven Durchmesser dieser Wendel hinausgeht, und daß die Öffnung (109) zwischen diesen Wendeln angeordnet ist.

6. Elektromagnetische Körperbehandlungsvorrichtung zur operativ nicht-invasiven Modifikation des Wachstums-, Wiederherstellungs- und Erhaltungsverhaltens von lebenden Geweben und Zellen durch eine spezifische sowie selektive Änderung in einer elektrischen Umgebung mit elektrischen Spuleneinrichtungen (115, 116), mit einer Halteeinrichtung (117), die die Spuleneinrichtungen in bezug auf eine erkrankte, zu behandelnde Körpergegend trägt, und mit einer elektrischen Impulserzeugereinrichtung, die die Spuleneinrichtungen mit einer Folge von Niederspannungsimpulsen erregt, dadurch gekennzeichnet, daß die Halteeinrichtung eine in Längsrichtung geschlitzte Röhre (117) aus nachgiebigem, unmagnetischen Material umfaßt, die vorübergehend im Verlauf des Anbringens an einem zu behandelnden Körperteil verformbar sowie im angebrachten Zustand in Umfangsrichtung kontinuierlich ist, und daß die Spuleneinrichtungen zwei gleiche Spulenwindung-Untergruppen umfassen, von denen jede für einen stirnseitigen Anbau an die jeweiligen Enden der Röhre ausgestaltet ist, wobei der Abstand im angebauten Zustand der Spulenwindung-Untergruppen geringer als ihr Durchmesser ist und flexible Einrichtungen (120) die Spulenwindung-Untergruppen für eine gleichzeitige Erregung in einer kraftflußunterstützenden Beziehung elektrisch verbinden.

7. Behandlungsvorrichtung nach Anspruch 6, dadurch gekennzeichnet, daß jede der Spulenwindung-Untergruppen eine Mehrzahl von Spulenwicklungen umfaßt, die in einem gehärteten, ringförmigen Körper aus einem unmagnetischen härtbaren Material eingebettet sind, der mit einer zylindrischen Einsenkung an einer Stirnseite für einen zusammenschiebbaren Anbau über einem Ende der Röhre versehen ist, wobei die innere Stirnfläche der zylindrischen Einsenkung einen den zusammenschiebbaren Anbau bei seinem Anstoßen an das Röhrenende begrenzenden Bund bestimmt.

**Revendications**

1. Dispositif pour le traitement électromagnétique du corps permettent de modifier, sans intervention d'ordre chirurgical, le comportement de croissance, de régénération et de conservation de tissus vivants et de cellules vivantes au moyen d'une modification spécifique et sélective de l'environnement électrique, incluant des moyens en forme de bobines électriques (86,86',93,94; 110,111,112), un cadre tubulaire rigide (80;90;l08) portant lesdits moyens en forme de bobines de manière à supporter ces derniers en rapport avec une partie malade du corps, devant être traitée, lesdits moyens en forme de bobines comportant des spires disposées autour d'un axe de développement du flux dans une zone de traitement destinée à recevoir la partie malade du corps, et des moyens en forme de générateur d'impulsions servant à exciter électriquement lesdits moyens en forme de bobines avec une succession d'impulsions à basse tension, caractérisé en ce que ledit cadre tubulaire rigide comporte une ouverture (81;91;l09) ménagée dans sa paroi tubulaire, qui permet d'introduire dans ledit élément en forme de cadre, tout en pouvant l'en retirer, un membre du corps, dans lequel se situe la partie malade, et de le faire passer dans l'ouverture de ce cadre de manière à placer la partie malade dans la zone de traitement desdits moyens en forme de bobines.

2. Dispositif de traitement selon la revendication 1, caractérisé en ce que lesdits moyens en forme de bobines comprennent deux bobines (86,86') montées en des emplacements diamétralement opposés dudit cadre tubulaire, et en ce que ladite ouverture est située à l'intérieur de l'une desdites bobines.

3. Dispositif de traitement selon la revendication 1, caractérisé en ce que lesdits moyens en forme de bobines comprennent deux bobines (93,94) montées en des emplacements diamétralement opposés dudit cadre tubulaire, et en ce que ladite ouverture est située entre lesdites bobines.

4. Dispositif de traitement selon la revendication 2 ou 3, caractérisé en ce que chacune desdites bobines possède une configuration de forme générale elliptique.

5. Dispositif de traitement selon la revendication 1, caractérisé en ce que lesdits moyens en forme de bobines comprennent au moins un enroulement possédant une pluralité de spires s'enroulant d'une manière générale selon une disposition en hélice autour de l'axe dudit cadre tubulaire, en ce que ladite pluralité de spires sont disposées sous la forme d'hélices (110,112) séparées axialement par une distance supérieure au diamètre effectif desdites spires, et en ce que ladite ouverture (109) est située entre lesdites hélices.

6. Dispositif pour le traitement électromagnétique du corps permettant de modifier, sans intervention d'ordre chirurgical, le comportement de croissance, de régénération et de conservation de tissus vivants et de cellules vivantes au moyen d'une modification spécifique et sélective de l'environnement électrique, comportant des moyens en forme de bobines électriques (115,116), des moyens de retenue (117) servant à supporter lesdits moyens en forme de bobines en rapport avec une partie malade du corps, devant être traitée, et des moyens formant générateur d'impulsions servant à exciter électriquement lesdits moyens en forme de bobines avec une succession d'impulsions à basse tension, caractérisé en ce que lesdits moyens de retenue comprennent un tube (117) fendu longitudinalement et réalisé en un matériau amagnétique élastique, qui peut se déformer transitoirement lors de sa mise en place sur un membre du corps devant être soumis à un traitement, et qui, lorsqu'il est ainsi mis en place, possède une configuration continue circonférentiellement, et en ce que lesdits moyens en forme de bobines comportent deux sous-ensembles identiques d'enroulements de bobines, dont chacun est adapté pour être monté sur les extrémités respectives dudit tube, l'espacement desdits sous-ensembles d'enroulements de bobines, à l'état assemblé, étant inférieur à leur diamètre, et des moyens flexibles (120) raccordant électriquement lesdits sous-ensembles d'enroulements de bobines pour permettre une excitation simultanée selon une relation favorisant l'établissement du flux.

7. Dispositif de traitement selon la revendication 6, caractérisé en ce que chacun desdits sous-ensembles d'enroulements de bobines comporte une pluralité de spires d'enroulement insérées dans un corps annulaire durci formé d'un matériau amagnétique durcissable comportant, sur une extrémité, un contre-perçage permettant son montage télescopique sur une extrémité dudit tube, l'extrémité intérieure du contre-percage définissant une bride servant à limiter l'assemblage télescopique sous l'effet de sa venue en butée contre l'extrémité du tube.

Fig.1

85

87

83

82

86

81

86

86'

80  86'  88

Fig. 7

112

109

111

110

108

Fig. 8

112

108

110

109

111

113

Fig. 9

116

115  119  118  117

120

Fig. 3.

Fig. 4.

Fig. 5.

Fig. 2.

Fig. 6.